(19) Europäisches Patentamt / European Patent Office / Office européen des brevets

(11) **EP 2 896 962 A1**

(12) **EUROPEAN PATENT APPLICATION**

(43) Date of publication:
**22.07.2015 Bulletin 2015/30**

(51) Int Cl.:
**G01N 27/407** (2006.01)    **G01N 33/00** (2006.01)

(21) Application number: **15000536.1**

(22) Date of filing: **24.02.2015**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Sensirion AG**
**8712 Stäfa (CH)**

(72) Inventors:
• **Streiff, Matthias**
  **CH-8712 Stäfa (CH)**
• **Rupp, Jennifer L.M.**
  **CH-8712 Stäfa (CH)**

(74) Representative: **Toleti, Martin**
  **c/o E.Blum & Co. AG**
  **Vorderberg 11**
  **8044 Zürich (CH)**

(54) **Gas sensor chip**

(57)    A gas sensor chip (1) comprises a substrate (2), a cavity (211) in the substrate, and a membrane (3) extending over the cavity (211). Furthermore, the gas sensor chip (1) comprises an arrangement comprising a thin-film solid material (4), wherein the thin-film solid material (4) is arranged between a reference electrode (5) and a sensing electrode (6), and a heating element (7) arranged in or on the membrane (3), for heating the thin-film solid material (4), the reference electrode (5), and the sensing electrode (6). The arrangement is arranged on a front side (31) of the membrane (3) or on a reverse side of the membrane (3) within the cavity (211), wherein the thin-film solid material (4) has a Li-ions conducting garnet-like crystal structure.

Fig. 1

EP 2 896 962 A1

**Description**

Technical Field

The present invention relates to a gas sensor chip and a method for manufacturing of a gas sensor chip.

Background Art

Gas sensors experience a rising demand for various applications, for instance for monitoring environmental conditions. Carbon dioxide, $CO_2$, is a gas which is particularly interesting for being monitored since it is a good indicator for environmental conditions or indoor air quality.

Sensing of carbon dioxide gas is also interesting in view of the emerging market of mobile devices, wherein such devices are increasingly equipped with complex sensing functionality, for instance with complimentary sensing functions, besides state-of-the-art humidity or temperature sensing functions. For example, such a functionality could be the monitoring of the environmental conditions, for which in particular the sensing of $CO_2$ gas is a basic premise.

Conventional gas sensors for $CO_2$ detection are based on spectroscopic measurement methods. For example, gas sensors using Non-Dispersive Infra Red (NDIR) absorption are used for detecting $CO_2$ in a gaseous environment by measuring its characteristic absorption spectrum. But NDIR sensors are large in size and in addition require elaborate manufacturing. Therefore, NDIR sensors are ineligible for integrated circuit applications and therefore ineligible for many applications, for instance ineligible for mobile device applications.

Disclosure of the Invention

The objective of the invention is to provide an improved gas sensor chip, particularly which provides sensing functionality on a small scale, and a method for the manufacturing thereof.

In a first aspect, the gas sensor chip comprises a substrate and a cavity in the substrate. The cavity can be arranged on a front side or on a reverse side of the substrate. A membrane extends over the cavity, preferably in order to form a hotplate. The membrane provides an inherent thermal insulation for the rest of the substrate comprising a CMOS circuit. The gas sensor chip further comprises an arrangement comprising a thin-film solid material arranged between a reference electrode and a sensing electrode. The arrangement is arranged on a front side of the membrane or on a reverse side of the membrane within the cavity. A heating element, e.g. a CMOS compatible micro-hotplate, is arranged in or on the membrane (wherein the term "arranged on" can include being arranged on the front side or on the reverse side of the membrane), for heating the thin-film solid material, the reference electrode, and the sensing electrode. The thin-film solid material has a Li-ions conducting garnet-like crystal structure, preferably a Li-ions conducting garnet-like crystal metal oxide structure. The thin-film solid material is also referred to as solid state material.

Garnets are orthosilicates of a general composition $X_3Y_2(ZO_4)_3$, which crystallize in a cubic crystal system, wherein X, Y and Z are sites in the crystal lattice. Compounds with the structure $Li_5La_3M_2O_{12}$, with M being a cation site, have a garnet-like structure. Li-ions conducting garnet-like crystal structures have the general formula, wherein they contain an excess of Li ions compared to ideal garnets and therefore exhibit Li-ions conductivity. Li-ions conducting garnet-like crystal structures are known, e.g. from WO 2005/08518 A1, page 2 to page 3. This document is incorporated by reference. Preferably, the Li-ions conducting garnet-like crystal structures known from WO 2013/010692 A1 are incorporated by reference.

Preferably, the thin-film solid material refers to a layer of solid material forming a thin-film layer of preferably less than 2 $\mu$m in film thickness, and very particular preferably less than 500 nm in film thickness. In a preferred embodiment, the thin-film solid material has a thickness of 10 to 300 nm. Further, the thin-film solid material preferably has a surface area of less than 0.25 mm$^2$.

Preferably, the arrangement of the chip comprises an electrochemical cell, comprising the thin-film solid material, the reference electrode and the sensing electrode. The thin-film solid material therefore can comprise a solid electrolyte, which is a predominantly good conductor for Li-ions and essentially blocking for electrons. The reference electrode and the sensing electrode can comprise mixed ionic-electronic conducting materials. A sensing functionality of the gas sensor chip is preferably based on an interaction of species from the electrochemical cell and a gas phase. Preferably, the gas sensor chip is a $CO_2$ gas sensor. A potential difference can originate from an interaction of a gas detected at the sensing electrode and injection of charge carriers to the thin-film solid material and reference electrode. The gas to be detected at the gas sensing electrode may be e.g. adsorbed $CO_2$.

Without being bound to theory it is believed, that the sensing reaction starts either with adsorption of a gas at a surface of the sensing electrode and/or at a boundary between the sensing electrode and the thin-film solid material. A reaction at the sensing electrode comprises a reaction of ions from the thin-film solid material, electrons from the sensing electrode, $CO_2$ from the gas phase and oxygen from a dissociated oxygen molecule in the gas phase, in a reaction step. Detection of $CO_2$ in the gas is characterized by a change in an electric potential difference over a first interface between the reference electrode and the thin-film solid material and a second interface between the sensing electrode and the thin-film solid material incorporating all bulk resistive material contributions as well.

The substrate can be a silicon substrate with CMOS circuitry integrated on the silicon substrate.

All electrodes and circuitry of the gas sensor chip may be formed by CMOS processing, and the building and/or arranging of the electrodes may be implemented in a way compatible to CMOS processing.

Also the membrane and/or the substrate and/or the thin-film solid material may be fabricated by CMOS manufacturing processes and/or may be compatible to CMOS processing. There may be electrical contacts between the electrodes and metallic layers of CMOS layers, in order to measure the potential difference.

A protection layer can be arranged between the heating element and the thin-film solid material for insulating the heating element. The heating element can comprise multiple heating elements. The heating element can heat the membrane within a range of room temperature (RT) up to an annealing temperature, e.g. of below 700°C. The heating element can therefore serve on one hand for annealing the thin-film solid material during manufacturing of the sensor and on the other hand for controlling the temperature of the membrane during operation of the sensor. During operation of the sensor, the heating temperature might be in a range of RT up to ca. 600°C. By increasing the heating temperature during operation of the gas sensor chip, the response time of the sensor can be increased (e.g. by operation at 500°C).

In a first embodiment of the invention the reference electrode may be arranged on the membrane, including the heating element. The thin-film solid material may be arranged on the reference electrode and can additionally also be partly arranged on the membrane. The sensing electrode may be arranged on the thin-film solid material or is in contact with the thin-film solid material but may be arranged such, that the sensing electrode and the reference electrode do not touch each other. The sensing electrode can form a cap, for protecting the thin-film solid material from environmental influences, wherein the cap covers all the thin-film solid material except for the side that adjoins the membrane, the reference electrode and/or the heater. Preferably, a distance between the reference electrode and the sensing electrode across the thin-film solid material is defined by a thickness of the thin-film solid material and is preferably less than 2 $\mu$m, very particular preferably less than 500 nm. Due to the short distance between the reference electrode and the sensing electrode, ohmic losses are strongly reduced.

In a second embodiment of the invention the thin-film solid material may be arranged on the membrane. A protecting layer may be arranged between the heating element and the thin-film solid material. The reference electrode and the sensing electrode preferably are arranged on opposite ends on the thin-film solid material. The opposite ends relate to opposite sides of the thin-film solid material, which are horizontally aligned in relation to the substrate.

In an additional embodiment, the arrangement is arranged on the front side of the membrane and a further arrangement, comprising a further thin-film solid material, wherein the further thin-film solid material is arranged between a further reference electrode and a further sensing electrode, is arranged on the reverse side of the membrane.

Preferably, in all embodiments, there are electrical contacts between the electrodes and a CMOS circuitry integrated in the substrate.

The reference electrode preferably comprises a metal, e.g. gold (Au), platinum (Pt), palladium (Pd), or aluminum (Al) or alloys thereof, or a metal oxide material, e.g. lithium titanate, lithium tungstenate, lithium manganate, lithium ferrite, strontium ruthenate or lanthanum nickelate. The reference electrode is preferably less than 2 $\mu$m thick. The reference electrode can also comprise tungsten (W).

In a further embodiment, the sensing electrode can comprise an auxiliary layer. In one embodiment, the sensing electrode comprises pure lithium carbonate. In a further embodiment, the sensing electrode comprises solid solutions of lithium carbonate with alkaline metals, such as Ba, Ca or Sr. The sensing electrode can be permeable to gas, e.g. can be a porous layer. Furthermore, the sensing electrode is preferably less than 5 $\mu$m thick.

In a further embodiment, the sensing electrode comprises surface structures, for increasing an available sensing electrode area per sensor volume for gas adsorption. E.g. cavities with feature length sizes of 5 nm to 100 $\mu$m in diameter can be structured into the sensing electrode. In an additional embodiment, the sensing electrode is structured, e.g. comprises holes or open pores, for increasing the available sensing electrode area per sensor volume for gas adsorption.

The Li-ions conducting garnet-like crystal structure can be of a formula (I)

$$\text{Li}_n[A_{(3-a'-a'')}A'_{(a')}A''_{(a'')}] [B_{(2-b'-b'')}B'_{(b')}B''_{(b'')}] [C'_{(c')}C''_{(c'')}]O_{12}, \qquad (I)$$

wherein A, A', and A'' represent a dodecahedral position of the Li-ions conducting garnet-like crystal structure, wherein

A represents at least one element selected from the group consisting of La, Y, Pr, Nd, Sm, Eu, Gd, Tb, Dy, Ho, Er, Tm and Yb;
A' represents at least one element selected from the group consisting of Ca, Sr and Ba;
A'' represents at least one element selected from the group consisting of Na and K; where

$$0 \leq a' < 2,$$

and

$$0 \leq a'' < 1,$$

wherein B, B' and B" represent an octahedral position of the Li-ions conducting garnet-like crystal structure, wherein
B represents at least one element selected from the group consisting of Zr, Hf, and Sn;
B' represents at least one element selected from the group consisting of Ta, Nb, Sb and Bi;
B" represents at least one element selected from the group consisting of Te, W, and Mo; where

$$0 \leq b' \leq 2,$$

and

$$0 \leq b'' \leq 2,$$

wherein C' and C" represent a tetrahedral position of the Li-ions conducting garnet-like crystal structure, wherein
C' represents at least one element selected from the group consisting of A1 and Ga;
C" represents at least one element selected from the group consisting of Si and Ge; where

$$0 \leq c' \leq 0.5,$$

and

$$0 \leq c'' \leq 0.4,$$

and
wherein

$$n = 7 + a' + 2*a'' - b' - 2*b'' - 3*c' - 4*c'',$$

and
wherein $5.5 \leq n \leq 6.875$,
wherein
if b' = 2, is $6.0 < n < 6.875$ or $5.5 \leq n \leq 6.875$ and c'+ c'' > 0, and
wherein
if B' is Nb, is $6.0 < n < 6.4$ or $5.5 \leq n \leq 6.875$ and c' + c" > 0 and/or a' + a" > 0.

In a further embodiment, the above Li-ions conducting garnet-like crystal structure of the thin-film solid material preferably has a stoichiometric composition with formula (II)

$$Li_{7-x} (La_3Zr) O_{12-y}, \qquad (II)$$

where

$$0 \leq x \leq 3,$$

and

$$0 \leq y \leq 3,$$

wherein x and y are rational numbers and wherein the thin-film solid material has a Li-ions conducting garnet-like crystal structure.

As becomes apparent from the above formulae (I) and (II), the Li-ions conducting garnet-like crystal structure comprises a pristine garnet-like structure, a doped garnet-like structure, a pristine garnet-like structure stabilized with Al, and/or a doped garnet-like structure stabilized with Al.

Accordingly, the Li-ions conducting garnet-like crystal structure can comprise dopants. Such dopants can be La, Y, Pr, Nd, Sm, Eu, Gd, Tb, Dy, Ho, Er, Tm, Yb, Ca, Sr, Ba, Na, K, Zr, Hf, Sn, Ta, Nb, Sb, Bi, Te, W, Mo, Al, Ga, Si, or Ge and combinations thereof. Preferred solid solution dopants, in particular for formula (II), can be Ta, Nb, Gd, Sm, Ce or Sr and combinations thereof.

Further, the Li-ions conducting garnet-like crystal structure can be stabilized, e.g. by comprising Al in grain boundaries.

The thin-film solid material defined herein might be processed at temperatures of below 700°C. It is believed that this is due to its thin-film characteristics.

A grain size of the thin-film solid material is defined by its average grain size of preferably below 150 nm, for fast reaction kinetics at the first interface between the reference electrode and the thin-film solid material and/or at the second interface between the sensing electrode and the thin-film solid material.

The average grain size refers to a diameter of individual grains of a microstructure of the thin-film solid material. A very common method of measuring the average grain size is to compare grains at a fixed magnification with grain size charts from the American Society for Testing and Materials. The average grain size of the thin-film solid material can be measured by X-ray diffraction and/or electron microscopy to be between 5 nm to 200 nm in average.

It is believed, that the thin-film characteristics and the small average grain size of the thin-film solid material lead to advantageous characteristics of the gas sensor chip. Due to the small average grain size, the gas sensor chip can provide a fast response time of a gas detecting signal. Also the high ionic conductivity of the thin-film solid material enables a fast response at low operation temperature and therefore can reduce an effective energy consumption of the heating element. In a preferred embodiment, the ionic conductivity of the thin-film solid material is at least $10^{-5}$ S/cm at 20°C. In addition, a small scale of the gas sensor chip enables its application in mobile devices.

In a further embodiment, the thin-film solid material has a stoichiometric composition $Li_{6.4}Ga_{0.2}La_3Zr_2O_{12}$, having a Li-ions conducting garnet-like crystal structure and a bulk ionic conductivity around $10^{-4}$ S/cm at 20°C and reveals phase stabilized by traceable aluminum additions.

A further aspect of the present invention is a method for manufacturing the gas sensor chip according to claim 1. A gas sensor chip comprising a substrate with a reverse side comprises a central region, which is etched down to a predefined thickness, for forming a cavity. A membrane at a front side of the substrate extends over the cavity. The membrane comprises preferably an oxide layer and in particular a silicon oxide ($SiO_2$) layer, a silicon nitride ($Si_3Ni_4$) layer, or an aluminium oxide ($Al_2O_3$) layer. The membrane can further comprise a part of the substrate. A heating element is arranged in or on the membrane (wherein "arranged on" includes being arranged in or on each side of the membrane). Etching can be performed by silicon bulk and surface-micromachining. The membrane can also be formed by using silicon-on-insulator (SOI) technology. An arrangement is manufactured on a front side and/or on a reverse side of the membrane. The arrangement comprises a thin-film solid material arranged between a reference electrode and a sensing electrode. The reference electrode and/or the sensing electrode and/or the thin-film solid material are fabricated on the substrate by means of thin-film processing methods. Preferably, the thin-film solid material is deposited at temperatures of below 450°C by pulsed laser deposition. Furthermore, the thin-film solid material is preferably annealed at temperatures of below 700°C, preferably by means of the heating element. Further thin-film processing methods include sputtering or any other vacuum or wet-chemical deposition methods. Preferably, the thin-film solid material is prepared from a pellet of the thin-film solid material by a vacuum-based technique for deposition.

Thereby, the components of the gas sensor chip can be of micro- and nanoscale size and can be fabricated by means of micro- and nanoscale fabrication methods.

Other advantageous embodiments are listed in the dependent claims as well as in the description below.

Brief Description of the Drawings

Embodiments of the present invention, aspects and advantages will become apparent from the following detailed description thereof. Such description makes reference to the annexed drawings, wherein the figures show:

Fig. 1 a schematic sectional view of a portion of a gas sensor chip with a cross-plane formation of an arrangement in accordance with an embodiment of the invention,

Fig. 2 a schematic sectional view of a portion of a gas sensor chip with a cross-plane formation of an arrangement in accordance with a further embodiment of the invention,

Fig. 3 is a schematic sectional view of a portion of a gas sensor chip with a cross-plane formation of an arrangement with a further embodiment of a sensing electrode in accordance with a further embodiment of the invention,

Fig. 4 is a schematic sectional view of a portion of a gas sensor chip with a cross-plane formation of an arrangement with a further embodiment of a sensing electrode in accordance with a further embodiment of the invention,

Fig. 5 is a schematic sectional view of a portion of a of gas sensor chip with an in-plane formation of an arrangement in accordance with an embodiment of the invention,

Fig. 6 is a schematic sectional view of a portion of a gas sensor chip with an in-plane formation of an arrangement in accordance with a further embodiment of the invention,

Fig. 7 is a schematic sectional view of a portion of a gas sensor chip with an arrangement on a reverse side of a membrane, in accordance with a further embodiment of the invention, and

Fig. 8 is a schematic sectional view of a portion of a gas sensor chip with an arrangement on a front side of a membrane and an additional arrangement on a reverse side of the membrane, in accordance with a further embodiment of the of the invention.

## Modes for Carrying Out the Invention

The embodiments of the invention comprise a substrate 2, which is preferably a silicon (Si) substrate with a front side and a reverse side 21. They further comprise a cavity 211 in the substrate 2 and a membrane 3, arranged at the front side of the substrate 2. The membrane 3 extends over the cavity 211 in order to form a hotplate. A heating element 7 is arranged in or on the membrane 3, wherein the heating element 7 can comprise one or more heating structures, which can be distributed in or on the membrane 3. The membrane 3 provides an inherent thermal insulation for the substrate 2 with a CMOS circuit. The temperature of the membrane 3 can rise rapidly wherein the temperature of the thicker substrate 2 reacts with a slower rise of temperature.

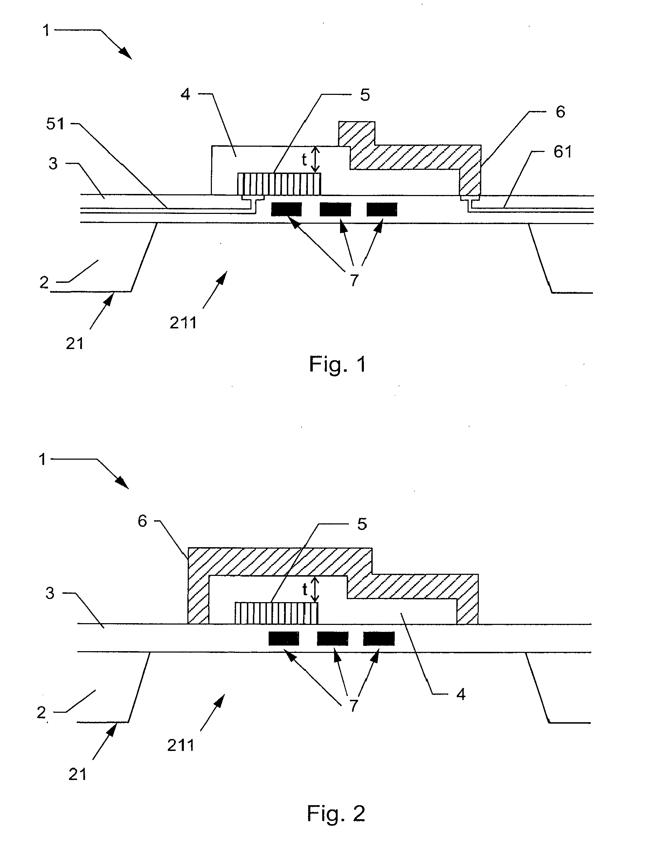

In particular, Fig. 1 shows a first embodiment of a portion of a gas sensor chip 1, with a cross-plane formation of an arrangement, comprising a thin-film solid material 4 between a reference electrode 5 and a sensing electrode 6. A distance t of the thin-film solid material 4 is defined as a thickness of the thin-film solid material 4. The reference electrode 5 is arranged on the membrane 3, wherein the membrane 3 includes the heating element 7. Preferably, a first connecting electrode 51 is arranged in or on the membrane 3, for connecting the reference electrode 5 e.g. via metallic CMOS layers to a signal processing circuit. The reference electrode 5 can also be partly integrated in the membrane 3.

A thin-film solid material 4 is arranged on the reference electrode 5, wherein the thin-film solid material 4 can be partly arranged on the membrane 3. There can be a protection layer between the heating element 7 and the reference electrode 5 and/or between the heating element 7 and the thin-film solid material 4.

A sensing electrode 6 is arranged on the thin-film solid material 4. The sensing electrode 6 only partially covers the thin-film solid material 4. A second connecting electrode 61 is arranged in or on the membrane 3, for connecting the sensing element 6 with the signal processing circuit.

Fig. 2 shows a further embodiment of a portion of a gas sensor chip 1, with a cross-plane formation of an arrangement, wherein the embodiment distinguishes from the embodiment of Fig. 1 by:

The sensing electrode 6 forms a cap for covering all the thin-film solid material 4 except for the side that adjoins the membrane 3 and/or the reference electrode 5, for protecting the thin-film solid material 4 from environmental influences. The sensing electrode 6 can comprise a porous layer, e.g. being permeable for a gas to be detected.

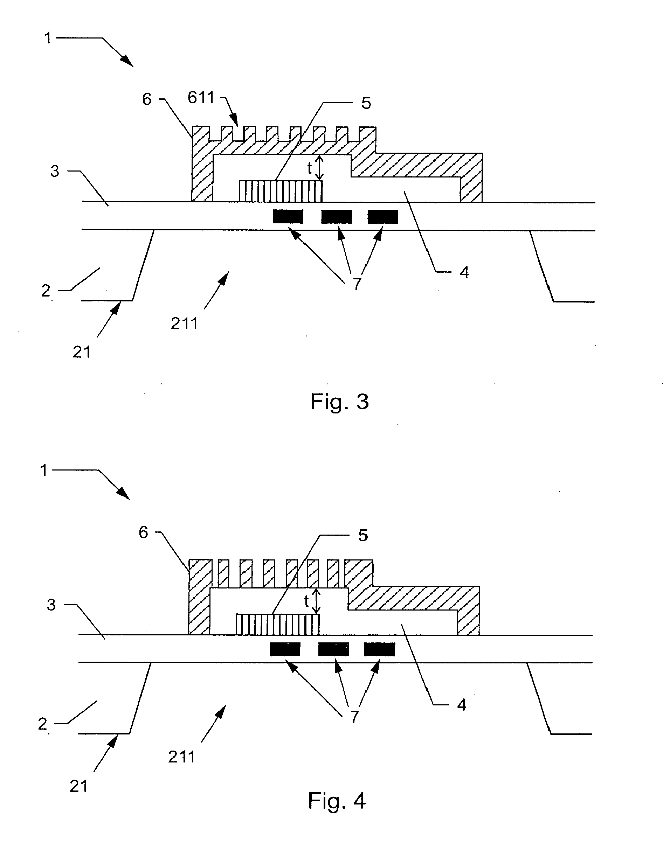

Fig. 3 shows a further embodiment of a portion of a gas sensor chip 1, with a cross-plane formation according to Fig. 1 or Fig. 2, wherein the embodiment distinguishes from the embodiments in Fig. 1 and Fig. 2 by:

The sensing electrode 6 comprises surface structures 611, for increasing an available sensing electrode area per sensor volume for gas adsorption. E.g. cavities with feature length sizes of 5 nm to 100 $\mu$m in diameter can be structured into the sensing electrode 6. Structuring can be done by lithography or non-lithography methods.

Fig. 4 shows a further embodiment of a portion of a gas sensor chip 1, which distinguishes from the embodiment in Fig. 1 or Fig. 2 or Fig. 3 by:

The sensing electrode 6 is structured, e.g. comprises holes or open pores, for increasing an available sensing electrode area per sensor volume for gas adsorption. A mean size of the structures, e.g. of the pores or holes, is in the range of 5 nm to 100 μm in diameter. The structures can be fabricated by lithography or non-lithography methods.

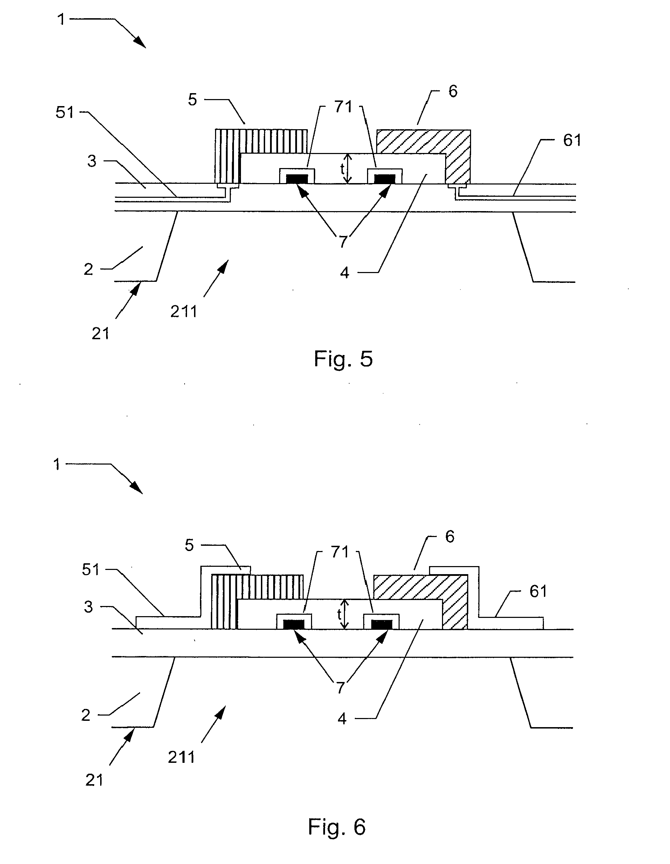

Fig. 5 shows a further embodiment of a portion of a gas sensor chip 1, with an in-plane formation of an arrangement, comprising a thin-film solid material 4 between a reference electrode 5 and a sensing electrode 6. The thin-film solid material 4 is arranged on the membrane 3, including the heating element 7, wherein the thin-film solid material 4 preferably covers a small area in the centre of the membrane 3. A distance t of the thin-film solid material 4 is defined as a thickness of the thin-film solid material. A reference electrode 5 and a sensing electrode 6 are arranged on opposite ends on the thin-film solid material 4. The opposite ends relate to opposite sides of the thin-film solid material, which are horizontally aligned in relation to the substrate. The reference electrode 5 and the sensing electrode 6 can be arranged such, that they are partly touching the membrane 3. In addition, there can be an insulating and/or protecting layer 71 between the thin-film solid material 4 and the heating element 7. In addition, also the reference electrode 5 can be covered with a protecting layer, for preventing contamination. Preferably, there are electrical contacts between the electrodes and the signal processing circuit. Preferably, a first connecting electrode 51 is arranged in or on the insulating layer 3, for connecting the reference electrode 5 with a signal processing circuit, which is preferably integrated on the gas sensor chip 1. In addition, a second connecting electrode 61 can be arranged in or on the insulating layer 3, for connecting the sensing electrode 6 with the signal processing circuit.

Fig. 6 shows a further embodiment of a portion of a gas sensor chip 1, with an in-plane formation of an arrangement, wherein the embodiment distinguishes from the embodiment of Fig. 5 by:

A first connecting electrode 51 is arranged on the reference electrode 5 and/or on the membrane 3, for connecting the reference electrode 5, e.g. via metallic CMOS layers, to the signal processing circuit. The first connecting electrode 51 can be partly arranged in the membrane 3.

A second connecting electrode 61 is arranged on the sensing electrode 6 and/or on the membrane 3, for connecting the sensing electrode 6, e.g. via metallic CMOS layers, to the signal processing circuit. The second connecting electrode 61 can be partly arranged in the membrane 3.

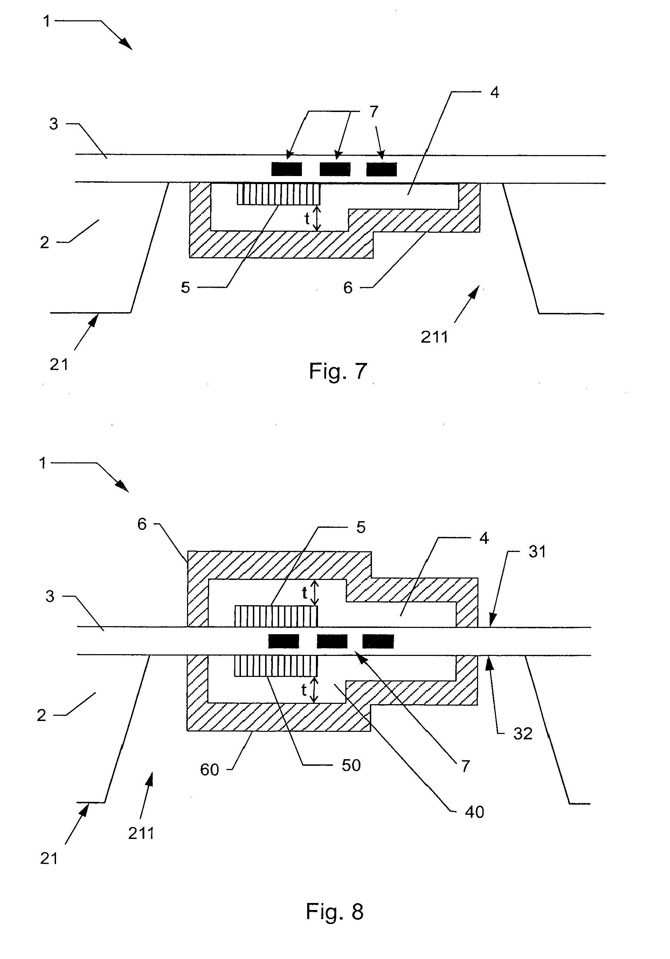

Fig. 7 shows a further embodiment of a portion of a gas sensor chip 1. An arrangement, comprising a thin-film solid material 4, arranged between a reference electrode 5 and a sensing electrode 6 is arranged on a reverse side 32 of the membrane 3, including the heating element 7, in the cavity 211. The cavity 211 can thereby comprise a gas to be detected. Preferably, there are electrical contacts between the electrodes (5, 6) and the signal processing circuit.

Fig. 8 shows a further embodiment of a portion of a gas sensor chip 1. An arrangement, comprising a thin-film solid material 4 between a reference electrode 5 and a sensing electrode 6, is arranged on a front side 31 of the membrane 3 and a further arrangement, comprising a further thin-film solid material 40 between a further reference electrode 50 and a further sensing electrode 60 is arranged on a reverse side 32 of the membrane 3 in the cavity 211. The reference electrode 5 can be partially integrated in the membrane 3. In this embodiment, one heating element 7 can serve for two arrangements. Preferably, there are electrical contacts between the electrodes and the signal processing circuit.

While there are shown and described presently preferred embodiments of the invention, it is to be distinctly understood that the invention is not limited thereto but may be otherwise variously embodied and practised within the scope of the following claims.

## Claims

1. A gas sensor chip (1), comprising
   a substrate (2),
   a cavity (211) in the substrate,
   a membrane (3) extending over the cavity (211),
   an arrangement comprising a thin-film solid material (4), wherein the thin-film solid material (4) is arranged between a reference electrode (5) and a sensing electrode (6),
   a heating element (7) arranged in or on the membrane (3), for heating the thin-film solid material (4), the reference electrode (5), and the sensing electrode (6),
   wherein the arrangement is arranged on a front side (31) of the membrane (3) or on a reverse side of the membrane (3) within the cavity (211),
   wherein the thin-film solid material (4) has a Li-ions conducting garnet-like crystal structure.

2. Gas sensor chip (1) according to claim 1,
   wherein the reference electrode (5) is arranged on the membrane (3),
   wherein the thin-film solid material (4) is arranged on the reference electrode (5) and/or on the membrane (3), and

wherein the sensing electrode (6) is arranged on the thin-film solid material (4) or is in contact with the thin-film solid material (4).

3. Gas sensor chip (1) according to claim 2,
wherein a distance between the reference electrode (5) and the sensing electrode (6) across the thin-film solid material (4) is less than 2 μm.

4. Gas sensor chip (1) according to claim 2 or 3,
wherein the sensing electrode forms a cap, for covering the thin-film solid material.

5. Gas sensor chip (1) according to claim 1,
wherein the thin-film solid material (4) is arranged on the membrane (3), and
wherein the reference electrode (5) and the sensing electrode (6) are arranged on opposite ends on the thin-film solid material (4).

6. Gas sensor chip (1) according to any one of the preceding claims,
wherein the arrangement is arranged on the front side (31) of the membrane (3), and/or
wherein a further arrangement, comprising a further thin-film solid material (40), wherein the further thin-film solid material (40) is arranged between a further reference electrode (50) and a further sensing electrode (60), is arranged on the reverse side (32) of the membrane (3) within the cavity (211).

7. Gas sensor chip (1) according to any one of the preceding claims,
wherein the reference electrode (5) comprises tungsten or another metal, in particular Au, Pt, Pd, or Al or alloys thereof, or comprises a metal oxide material, in particular lithium titanate, lithium tungstenate, lithium manganate, lithium ferrite, strontium ruthenate or lanthanum nickelate.

8. Gas sensor chip (1) according to any one of the preceding claims,
wherein the sensing electrode (6) comprises pure lithium carbonate or solid solutions of lithium carbonate with alkaline metals in particular Ba, Ca or Sr.

9. Gas sensor chip (1) according to any one of the preceding claims,
wherein the sensing electrode (6) comprises surface structures (611), for increasing the sensing area per sensor volume for gas adsorption, and/or
wherein the sensing electrode (6) is structured, preferably comprising holes and/or open pores, for increasing the available sensing electrode area per sensor volume for gas adsorption, and/or
wherein the sensing electrode (6) comprises a porous material.

10. Gas sensor chip (1) according to any one of the preceding claims, wherein
the thin-film solid material (4) is less than 2 μm in thickness; and/or
the sensing electrode is less than 5 μm thick; and/or
the reference electrode is less than 2 μm thick; and/or
the thin-film solid material has a surface area of less than 0.25 $mm^2$.

11. Gas sensor chip (1) according to any one of the previous claims,
wherein the Li-ions conducting garnet-like crystal structure is selected from
a pristine garnet-like structure; or
doped garnet-like structure; or
pristine garnet-like structure stabilized with Al; or
doped garnet-like structure stabilized with Al,
wherein the dopants are selected from the list of La, Y, Pr, Nd, Sm, Eu, Gd, Tb, Dy, Ho, Er, Tm, Yb, Ca, Sr, Ba, Na, K, Zr, Hf, Sn, Ta, Nb, Sb, Bi, Te, W, Mo, Al, Ga, Si, or Ge, and combinations thereof, and
preferably are selected from the list of Ta, Nb, Gd, Sm, Ce, or Sr and combinations thereof.

12. Gas sensor chip (1) according to any one of the previous claims,
wherein the Li-ions conducting garnet-like crystal structure is of a formula (II)
$Li_{7-x}(La_3Zr_2)O_{12-}y$, (II) where

$$0 \leq x \leq 3,$$

and

$$0 \leq y \leq 3,$$

wherein x and y are rational numbers.

13. Gas sensor chip (1) according to any one of previous claims,
wherein the thin-film solid material (4) has a bulk ionic conductivity of at least $10^{-5}$ S/cm at 20°C.

14. Gas sensor chip (1) according to any one of the previous claims,
wherein a grain size of the thin-film solid material (4) is below 150 nm.

15. A method for manufacturing the gas sensor chip (1) according to any one of claim 1 to claim 14,
wherein a central region of the substrate (2) is etched down to a predefined thickness, for forming the cavity (211), and/or
wherein the reference electrode (5) and/or the sensing electrode (6) and/or the thin-film solid material (4) are fabricated on the membrane (3) by means of thin-film processing methods.

16. Method for manufacturing the gas sensor chip (1) according to claim 15,
wherein the thin-film solid material (4) is deposited at temperatures below 450°C preferentially by pulsed laser deposition, and/or
wherein the thin-film solid material (4) is annealed at temperatures of below 700°C, preferably by means of the heating element (7).

Fig. 1

Fig. 2

Fig. 3

Fig. 4

Fig. 5

Fig. 6

Fig. 7

Fig. 8

Europäisches
Patentamt

European
Patent Office

Office européen
des brevets

## EUROPEAN SEARCH REPORT

Application Number

EP 15 00 0536

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>Y | US 2013/075255 A1 (MOON SEUNG EON [KR] ET AL) 28 March 2013 (2013-03-28)<br>* abstract *<br>* figures *<br>* paragraphs [0022] - [0049] *<br>----- | 1-11,15,16<br>12-14 | INV.<br>G01N27/407<br>G01N33/00 |
| X | JP 2013 174627 A (TDK CORP) 5 September 2013 (2013-09-05)<br>* abstract *<br>* figures *<br>* paragraphs [0016] - [0021] *<br>----- | 1,15 | |
| Y | ADAM RAMZY ET AL: "Tailor-Made Development of Fast Li Ion Conducting Garnet-Like Solid Electrolytes",<br>ACS APPLIED MATERIALS & INTERFACES,<br>vol. 2, no. 2,<br>24 February 2010 (2010-02-24), pages 385-390, XP055194723,<br>ISSN: 1944-8244, DOI: 10.1021/am900643t<br>* the whole document *<br>----- | 12-14 | |
| A | YOON JUNRO ET AL: "Interface reaction and its effect on the performance of a CO2 gas sensor based on Li0.35La0.55TiO3 electrolyte and Li2CO3 sensing electrode",<br>SENSORS AND ACTUATORS B: CHEMICAL: INTERNATIONAL JOURNAL DEVOTED TO RESEARCH AND DEVELOPMENT OF PHYSICAL AND CHEMICAL TRANSDUCERS, ELSEVIER S.A, CH,<br>vol. 182, 6 March 2013 (2013-03-06), pages 95-103, XP028533576,<br>ISSN: 0925-4005, DOI: 10.1016/J.SNB.2013.02.104<br>* abstract *<br>* page 97, column 2, line 1 - line 14 *<br>----- | 1-16 | |

TECHNICAL FIELDS SEARCHED (IPC)

G01N

-/--

The present search report has been drawn up for all claims

| Place of search | Date of completion of the search | Examiner |
|---|---|---|
| Munich | 11 June 2015 | Ruchaud, Nicolas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

## EUROPEAN SEARCH REPORT

Application Number

EP 15 00 0536

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | MAZZA D ET AL: "Modeling Li-ion conductivity in fast ionic conductor La2/3-xLi3xTiO3", SOLID STATE IONICS, NORTH HOLLAND PUB. COMPANY. AMSTERDAM; NL, NL, vol. 149, no. 1-2, 1 July 2002 (2002-07-01), pages 81-88, XP004360693, ISSN: 0167-2738, DOI: 10.1016/S0167-2738(02)00100-5 * abstract * * paragraph "1. Introduction" * * figure 1 * | 1-16 | |
| A | YAOJUN A. DU ET AL: "Li Ion Diffusion Mechanisms in the Crystalline Electrolyte [gamma]-Li3PO4", JOURNAL OF THE ELECTROCHEMICAL SOCIETY, vol. 154, no. 11, 1 January 2007 (2007-01-01), pages A999-A1004, XP055194659, ISSN: 0013-4651, DOI: 10.1149/1.2772200 * abstract * * figures 1,5 * * page A999, column 1, line 21, line 28 * * page A1001, column 1, line 28, column 2, line 7 * | 1-16 | |

-----

-/--

TECHNICAL FIELDS
SEARCHED      (IPC)

The present search report has been drawn up for all claims

| Place of search | Date of completion of the search | Examiner |
|---|---|---|
| Munich | 11 June 2015 | Ruchaud, Nicolas |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

Europäisches
Patentamt

European
Patent Office

Office européen
des brevets

# EUROPEAN SEARCH REPORT

Application Number

EP 15 00 0536

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | CHEN KAI ET AL: "Improving ionic conductivity of Li0.35La0.55TiO3 ceramics by introducing Li7La3Zr2O12 sol into the precursor powder", SOLID STATE IONICS, NORTH HOLLAND PUB. COMPANY. AMSTERDAM; NL, NL, vol. 235, 14 February 2013 (2013-02-14), pages 8-13, XP028986342, ISSN: 0167-2738, DOI: 10.1016/J.SSI.2013.01.007 * the whole document * | 1-16 | |
| A | US 2011/226042 A1 (YU JUNG KN [KR] ET AL) 22 September 2011 (2011-09-22) * abstract * * paragraph [0045] * | 1-16 | |
| A | LEE JAE-MYUNG ET AL: "High lithium ion conductivity of Li7La3Zr2O12 synthesized by solid state reaction", SOLID STATE IONICS, vol. 258, 16 February 2014 (2014-02-16), pages 13-17, XP028632653, ISSN: 0167-2738, DOI: 10.1016/J.SSI.2014.01.043 * abstract * | 1-16 | TECHNICAL FIELDS SEARCHED (IPC) |

The present search report has been drawn up for all claims

| Place of search | Date of completion of the search | Examiner |
|---|---|---|
| Munich | 11 June 2015 | Ruchaud, Nicolas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**                          EP 15 00 0536

This annex lists the patent family members relating to the patent documents cited in the above-mentioned European search report.
The members are as contained in the European Patent Office EDP file on
The European Patent Office is in no way liable for these particulars which are merely given for the purpose of information.

11-06-2015

| Patent document cited in search report | | Publication date | Patent family member(s) | | Publication date |
|---|---|---|---|---|---|
| US 2013075255 | A1 | 28-03-2013 | KR  20130034337 A<br>US  2013075255 A1 | | 05-04-2013<br>28-03-2013 |
| JP 2013174627 | A | 05-09-2013 | JP     5672339 B2<br>JP  2013174627 A | | 18-02-2015<br>05-09-2013 |
| US 2011226042 | A1 | 22-09-2011 | KR  20100064479 A<br>US  2011226042 A1<br>WO  2010064868 A2 | | 15-06-2010<br>22-09-2011<br>10-06-2010 |

For more details about this annex : see Official Journal of the European Patent Office, No. 12/82

**EP 2 896 962 A1**

**Patent documents cited in the description**

- WO 200508518 A1
- WO 2013010692 A1